# EUROPEAN PATENT SPECIFICATION

(11) **EP 2 948 110 B1**
(45) Date of publication and mention of the grant of the patent: **27.03.2019**
(21) Application number: 14742749.6
(22) Date of filing: 23.01.2014
(51) Int. Cl.: A61F 5/56

(54) **ORAL APPLIANCE**
ORALE ANWENDUNG
APPAREIL BUCCAL

(30) Priority: 23.01.2013 KR 20130007414; 10.12.2013 KR 20130153414
(43) Date of publication of application: 02.12.2015
(73) Proprietor: Choi, Hyun-Jin, Incheon 406-840 (KR); Park, Young-Hyon, Incheon 406-840 (KR)
(72) Inventor: PARK, Young-Hyon, Incheon 406-840 (KR); PARK, Jun-Young, Incheon 406-840 (KR)
(74) Representative: Stolmár & Partner Patentanwälte PartG mbB
(86) International application number: PCT/KR2014/000681
(87) International publication number: WO 2014/116044

(56) References cited:
- EP-A1- 1 092 403
- JP-A- 2010 029 615
- KR-B1- 101 133 537
- KR-B1- 101 218 036
- KR-B1- 101 218 036
- US-A- 5 499 633
- US-A1- 2012 060 848

## Description

### Technical Field

The present disclosure relates to an oral appliance installed in teeth arrangements of upper and lower jaws in an oral cavity and allowing a lower jaw to move forward by a predetermined distance to maintain an open state of airway, and more particularly to an oral appliance in which at least one of an upper teeth recess and a lower teeth recess is spaced apart from teeth inserted therein at a predetermined interval in vertical and horizontal directions.

### Background Art

During sleep, a tongue is hung backward within an oral cavity, and which gives a pressure on a airway to become narrowed.

In this state, inhaled air may not entirely enter the narrowed airway and instead remain in the oral cavity. Also, residual air vibrates soft tissues around the airway to generate noise, which is namely known as snoring.

Snoring may be frequently accompanied by obstructive sleep apnea.

In order to prevent snoring and sleep apnea, a CPAP (Constant Positive Airway Press device) forcibly supplying air to the airway during sleep may be used. However, while such a CPAP may be effective for snoring and sleep apnea, it is inconvenient to use and costly, and the CPAP somtimes evokes side effects.

Also, a surgical intervention to remove drooped soft tissues around such as palatopharynx, pharynx and soft palate may be considered, but this is not favorably accepted because recurrence is common and expected side effects.

Besides, there is an oral appliance installed in teeth arrangements of upper and lower jaws within an oral cavity to prevent snoring and sleep apnea. While the lower jaw to be maintained forcibly forward by the oral appliance, a tongue attached to the lower jaw is forwardly pulled out, thereby relieving pressure applied to the airway by the tongue and widening the airway to allow air to flow smoothly.

The oral appliance includes an integrated type oral appliance and an separated type oral appliance.

The integrated type oral appliance is formed by integrating an upper jaw teeth insertion part into which teeth included in a teeth arrangement of an upper jaw are inserted and a lower jaw teeth insertion part into which teeth included in a teeth arrangement of a lower jaw are inserted.

Also, the separated type oral appliance is formed by separating the upper jaw teeth insertion part and the lower jaw teeth insertion part.

The integrated type oral appliance has the upper jaw teeth insertion part and lower jaw teeth insertion part integrated, as mentioned above.

Thus, when the integrated type oral appliance is used, the lower jaw may not move after the lower jaw moves forward in a predetermined distance by the integrated type oral appliance during sleeping. Thus, a TMJ (Temporo-Mandibular Joint), neighboring muscles or a ligaments may be stiffened or pain may be present.

In addition, in the case of the separated type oral appliance, the upper jaw teeth insertion part and the lower jaw teeth insertion part are separated to allow the lower jaw to move during sleep, as mentioned above. Also, the upper jaw teeth insertion part and the lower jaw teeth insertion part are connected by a connection member to allow the lower jaw to move.

However, even with the separated type oral appliance, a movement of the lower jaw is restrained by the connection member, and the aforementioned problem remains unsolved. Document US 5,499,633 discloses an oral appliance comprising a body with an upper teeth recess and a lower teeth recess, configured so that the lower jaw is positioned into a more forward position compared to the upper jaw.

### Disclosure of Invention

### Technical problem

The present disclosure has been devised through recognition of any one of the issues or problems evident in the related art.

An aspect of the present disclosure provides an oral appliance allowing a lower jaw to move even if a user is wearing the oral appliance.

An aspect of the present disclosure also provides an oral appliance allowing stiffness of related muscles and ligaments, caused by a forward movement of a lower jaw, to be relaxed by enabling a lower jaw to move.

An aspect of the present disclosure also provides an oral appliance capable of preventing pain, malocclusion, or a TMJ disorder from occurring even while a user is wearing the oral appliance.

### Solution to Problem

The above-mentioned object is achieved by the teaching of the independent claims.

An oral appliance, in relation to an exemplary embodiment for performing at least one of the foregoing tasks, may have the following features.

According to an aspect of the present disclosure, there is provided an oral appliance including: a body having a shape corresponding to a teeth arrangement; an upper teeth recess formed in an upper portion of the body to allow teeth included in the teeth arrangement of an upper jaw to be inserted therein; and a lower teeth recess formed in a lower portion of the body to allow teeth included in the teeth arrangement of a lower jaw to be inserted therein and allowing the lower jaw to move forward by a predetermined distance.

The oral appliance may further include a guide part extending from the body in at least one or more of upward and downward directions by a predetermined distance to thereby guide teeth of the lower jaw or upper jaw to be inserted into the upper teeth recess or the lower teeth recess.

The guide part may extend from an inner side of the body.

The guide part may extend to cover at least a portion of a gum part of molars among the teeth.

The teeth of the upper jaw may be fixed in the upper teeth recess through friction, and the lower teeth recess may be spaced apart from the teeth of the lower jaw at predetermined intervals in vertical and horizontal directions.

A gap between the lower teeth recess and the teeth of the lower jaw in the vertical direction may range from 0.5 mm to 2 mm.

A gap between the lower teeth recess and the teeth of the lower jaw in the horizontal direction in one side thereof may range from 0.3 mm to 0.5 mm.

The teeth of the lower jaw may be fixed in the lower teeth recess through friction, and the upper teeth recess may be spaced apart from the teeth of the upper jaw at predetermined intervals in vertical and horizontal directions.

A gap between the upper teeth recess and the teeth of the upper jaw in the vertical direction may range from 0.5 mm to 2 mm.

A gap between the upper teeth recess and the teeth of the upper jaw in the horizontal direction in one side thereof may range from 0.3 mm to 0.5 mm.

The upper teeth recess may be spaced apart from the teeth of the upper jaw at a predetermined interval in vertical and horizontal directions, and the lower teeth recess may be spaced apart from the teeth of the lower jaw at a predetermined interval in vertical and horizontal directions.

A gap between the upper teeth recess and the teeth of the upper jaw in the vertical direction and a gap between the lower teeth recess and the teeth of the lower jaw in the vertical direction may range from 0.5 mm to 2 mm, and a gap between the upper teeth recess and the teeth of the upper jaw in the horizontal direction in one side thereof and a gap between the lower teeth recess and the teeth of the lower jaw in the horizontal direction in one side thereof may range from 0.3 mm to 0.5 mm.

### Advantageous Effects of Invention

As described above, according to an exemplary embodiment of the present disclosure, since a space is formed between an upper teeth recess or a lower teeth recess, to which teeth are inserted, and the teeth in vertical and horizontal directions in the oral appliance, when a user wears the oral appliance, a lower jaw may move.

Also, according to an exemplary embodiment of the present disclosure, stiffness of related muscles and ligaments caused by a forward movement of the lower jaw may be relaxed as a movement of the lower jaw is allowed.

In addition, according to an exemplary embodiment of the present disclosure, even if the oral appliance is worn, pain, malocclusion, or a TMJ disorder does not occur, unlike the related art oral appliance.

Moreover, according to an exemplary embodiment of the present disclosure, since air flow through an airway is facilitated, rhinitis or rhinosinusitis may be mitigated.

### Brief Description of Drawings

FIG. 1 includes perspective views of an oral appliance according to a first exemplary embodiment of the present disclosure, in which (a) is a top perspective view and (b) is a bottom perspective view.
FIG. 2 is a cross-sectional view taken along line A-A'in (a) of FIG. 1.
FIG. 3 is a view illustrating the oral appliance worn in an oral cavity according to the first exemplary embodiment of the present disclosure, in which (a) is a front view and (b) is a side view.
FIG. 4 is a cross-sectional view and a partially enlarged cross-sectional view of (b) of FIG. 3.
FIG. 5 is a cross-sectional view and a partially enlarged cross-sectional view illustrating a usage state of the oral appliance according to the first exemplary embodiment of the present disclosure.
FIG. 6 is a cross-sectional view and a partially enlarged cross-sectional view illustrating a wearing state of the oral appliance according to a second exemplary embodiment of the present disclosure.
FIG. 7 is a cross-sectional view and a partially enlarged cross-sectional view illustrating a wearing state of the oral appliance according to a third exemplary embodiment of the present disclosure.
FIG. 8 is a perspective cross-sectional view illustrating an oral appliance according to a further example of the present disclosure.

### Mode for the Invention

To help understand the foregoing features of the present disclosure, an oral appliance related to an exemplary embodiment of the present disclosure will be described in detail

Exemplary embodiments of the present disclosure will now be described in detail with reference to the accompanying drawings.

The disclosure may, however, be exemplified in many different forms and should not be construed as being limited to the specific embodiments set forth herein. Rather, these embodiments are provided so that this disclosure will be thorough and complete, and will fully convey the scope of the disclosure to those skilled in the art.

In the drawings, the shapes and dimensions of elements may be exaggerated for clarity, and the same reference numerals will be used throughout to designate the same or like elements.

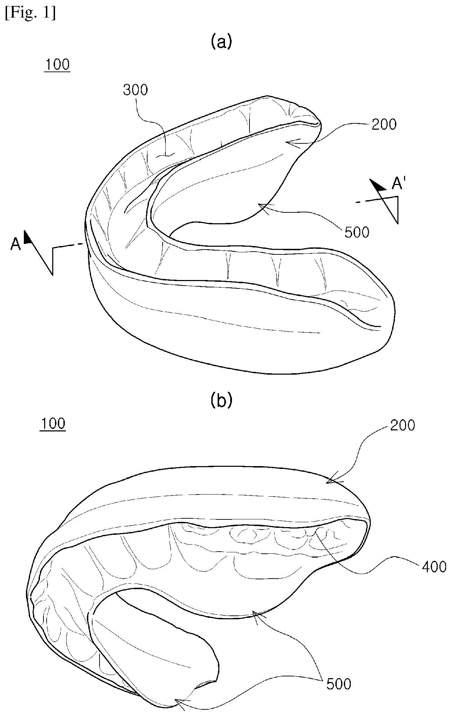

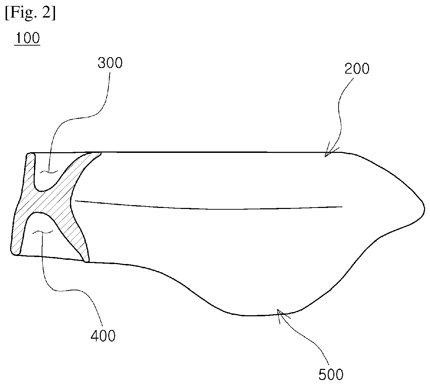

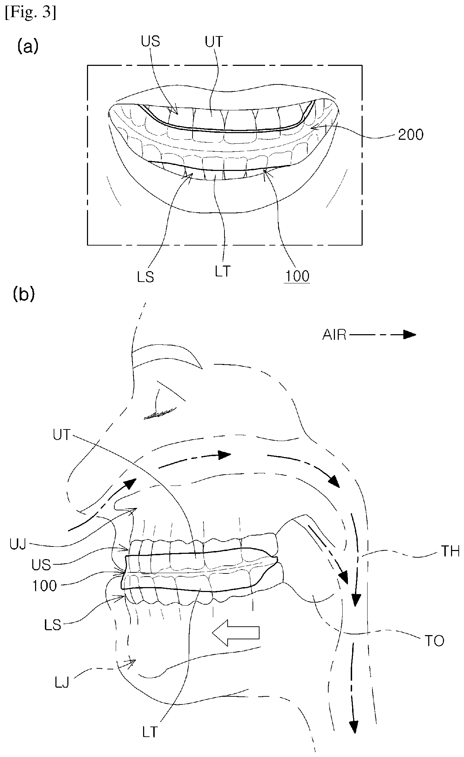

FIG. 1 includes perspective views of an oral appliance according to a first exemplary embodiment of the present disclosure, in which (a) is a top perspective view and (b) is a bottom perspective view, FIG. 2 is a cross-sectional view taken along line A-A' in (a) of FIG. 1, and FIG. 3 is a view illustrating the oral appliance worn in an oral cavity according to the first exemplary embodiment of the present disclosure, in which (a) is a front view and (b) is a side view.

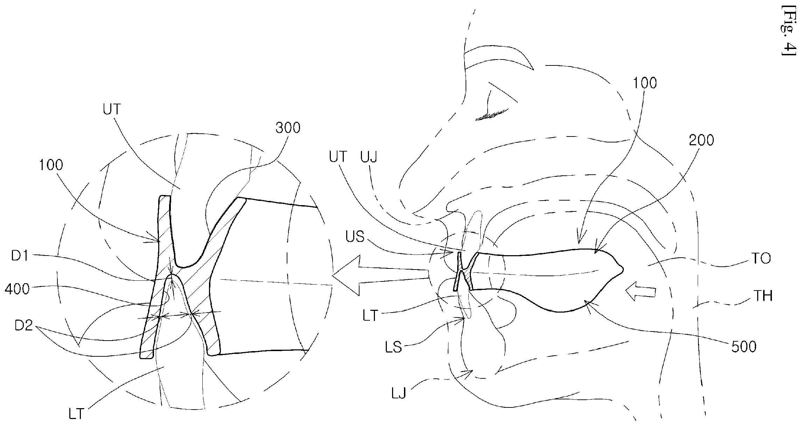

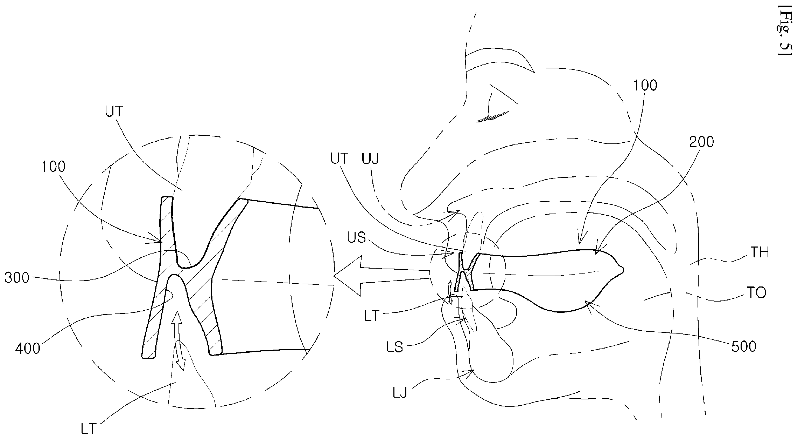

FIG. 4 is a cross-sectional view and a partially enlarged cross-sectional view of (b) of FIG. 3, and FIG. 5 is a cross-sectional view and a partially enlarged cross-sectional view illustrating a usage state of the oral appliance according to the first exemplary embodiment of the present disclosure.

An oral appliance according to the exemplary embodiment of the present disclosure may include a body 200, an upper teeth recess 300, a lower teeth recess 400, and a guide part 500.

As illustrated in FIG. 1, the body 200 may have a shape corresponding to teeth arrangements US and LS. Namely, the body 200 may have a horseshoe shape. Thus, the teeth arrangement US of an upper jaw UJ and the teeth arrangement LS of a lower jaw LJ may correspond to the body 200.

As illustrated in FIGS. 3 and 4, teeth UT included in the teeth arrangement US of the upper jaw UJ may be inserted into the upper teeth recess 300 formed in an upper portion of the body 200 as described hereinafter, and teeth LT included in the teeth arrangement LS of the lower jaw LJ may be inserted into the lower teeth recess 400 formed in a lower portion of the body 200 as described hereinafter.

A material of the body 200 is not particularly limited and may be any material as long as it does not pose a threat when the body 200 formed of the material is applied to an oral cavity.

In order to manufacture the oral appliance 100 according to an exemplary embodiment of the present disclosure including the body 200, any known method, such as forming a model of the teeth UT and LT of a user and subsequently shaping the oral appliance 100 appropriately, or manufacturing the oral appliance 100 by using a three-dimensional (3D) printer, or the like, may be used.

The upper teeth recess 300 may be formed in an upper portion of the body 200. As illustrated in FIGS. 3 through 5, the teeth UT included in the teeth arrangement US of the upper jaw UJ may be inserted into the upper teeth recess 300. To this end, the upper teeth recess 300 may have a shape corresponding to the teeth arrangement US of the upper jaw UJ on the whole, namely, a horseshoe shape as mentioned above; also, the upper teeth recess 300 may have a shape corresponding to each tooth UT included in the teeth arrangement US of the upper jaw UJ.

A method for forming the upper teeth recess 300 in the upper portion of the body 200 is not particularly limited, and any method, such as forming a model of the teeth UT of a user and subsequently shaping the upper teeth recess 300 to correspond thereto or forming the upper teeth recess 300 by using a 3D printer, or the like, may be used.

The teeth UT of the upper jaw UJ may be fixed to the upper teeth recess 300 through friction. Namely, as mentioned above, the upper teeth recess 300 may be formed to have a shape corresponding to the teeth UT of the upper jaw UJ such that there is no gap between the upper teeth recess 300 and the teeth UT of the upper jaw UJ.

Thus, the teeth UT of the upper jaw UJ may be tightly inserted into the upper teeth recess 300 and fixed to the upper teeth recess 300 through friction. Unless external force stronger than frictional force between the upper teeth recess 300 and the teeth UT of the upper jaw UJ is applied, the teeth UT of the upper jaw UJ may not be separated from the upper teeth recess 300.

The lower teeth recess 400 may be formed in a lower portion of the body 200. As illustrated in FIGS. 3 through 5, the teeth LT included in the teeth arrangement LS of the lower jaw LJ may be inserted into the lower teeth recess 400. To this end, the lower teeth recess 400 may have a shape corresponding to the teeth arrangement LS of the lower jaw LJ on the whole, namely, a horseshoe shape as mentioned above; also, the lower teeth recess 400 may have a shape corresponding to each tooth LT included in the teeth arrangement LS of the lower jaw LJ.

A method for forming the lower teeth recess 400 in the lower portion of the body 200 is not particularly limited, and any method, such as forming a model of the teeth LT of a user and subsequently shaping the lower teeth recess 400 to correspond thereto or forming the lower teeth recess 400 by using a 3D printer, or the like, may be used.

When the teeth LT of the lower jaw LJ is inserted into the lower teeth recess 400, the lower jaw LJ may move forward by a predetermined distance.

Namely, as illustrated in FIGS. 3 and 4, the lower jaw LJ may move forward by a predetermined distance such that front teeth of the lower jaw LJ are positioned in front of the front teeth of the upper jaw UJ by a predetermined distance. Accordingly, a tongue TO attached to the lower jaw LJ may also move forward along with the lower jaw LJ, as illustrated.

Thus, as illustrated in FIGS. 3 and 4, the tongue TO may not block the airway TH and the airway TH may also be widened. Also, as illustrated in (b) of FIG. 3, air may flow smoothly through the airway TH. Thus, snoring, occurring due to the tongue TO blocking the airway TH and the airway being narrowed during sleep, may be prevented. Also, sleep apnea occurring together with snoring may be prevented. Also, rhinitis or rhinosinusitis may be mitigated.

To this end, as mentioned above, the lower teeth recess 400 may be formed in the body 400 such that when the teeth LT of the lower jaw LJ is inserted, the teeth LT of the lower jaw LJ move forward relative to a natural position, such that the front teeth of the lower jaw LJ are positioned in front of the front teeth of the upper jaw UJ by a predetermined distance.

Meanwhile, as illustrated in FIG. 4, when the teeth LT of the lower jaw LJ are inserted into the lower teeth recess 400, the teeth LT of the lower jaw LJ may be spaced apart from the lower teeth recess 400 at predetermined gaps D1 and D2 therebetween in vertical and horizontal directions.

With the presence of the gaps D1 and D2 between the lower teeth recess 400 and the teeth LT of the lower jaw LJ in the vertical and horizontal directions, as illustrated in FIG. 5, the teeth LT of the lower jaw LJ may be released from the lower teeth recess 400 and moved downwards as needed; accordingly, the lower jaw LJ may also be moved downwards.

Thus, since the lower jaw LJ may naturally move downwards, stiffness of the neighboring muscles and ligaments occurred by, for example, the forward movement of the lower jaw LJ and maintenance of this state during sleep, may be resolved and relaxed.

The foregoing downward movement of the lower jaw LJ may be stopped when the guide part 500 comes into contact with a lingual mucous membrane of the lower jaw LJ. When this state is maintained, the neighboring muscles and ligaments may stiffen again.

In order to resolve this, the lower jaw LJ may naturally move upwards, namely, may return to a position before the movement, in which the teeth LT of the lower jaw LJ are inserted into the lower teeth recess 400 and the lower jaw LJ moves forward.

The downward movement of the lower jaw LJ from the oral appliance 100 and the upward movement thereof to the oral appliance 100 may be repeated while the user, wearing the oral appliance 100 according to the exemplary embodiment of the present disclosure, is sleeping.

Thus, even if the user wears the oral appliance 100 according to the exemplary embodiment of the present disclosure for an extended period of time in his or her sleep every night, the muscles and ligaments around the oral cavity may not be stiffened. Also, pain, malocclusion, or a TMJ disorder caused by rigidity of the muscles and ligaments around the oral cavity may not occur. Also, since air flow through the airway is improved, rhinitis or rhinosinusitis may be mitigated.

The gap D1 between the teeth LT of the lower jaw LJ and the lower teeth recess 400 in the vertical direction may range from 0.5 mm to 2 mm.

According to released research, a natural gap between the teeth UT of the upper jaw UJ and the teeth LJ of the lower jaw LJ, for example, the gap during sleep, ranges approximately from 2 mm to 4 mm.

Meanwhile, a distance between the upper teeth recess 300 and the lower teeth recess 400 is approximately 1.5 mm. Thus, if the gap D1 between the lower teeth recess 400 and the teeth LT of the lower jaw LJ in the vertical direction is smaller than 0.5 mm, a distance between the teeth UT of the upper jaw UJ and the teeth LT of the lower jaw may be smaller than 2 mm to 4 mm, the natural gap.

In this case, the brain may misunderstand the body 200 interposed between the teeth UT of the upper jaw UJ and the teeth LT of the lower jaw LJ to be food, causing masticatory movements to be made by the related muscles and ligaments. Force exerted by the masticatory movements may be transmitted to the teeth UT and LT of the upper and lower jaws UJ and LJ through the oral appliance 100 according to the exemplary embodiment of the present disclosure.

The force transmitted to the teeth UT and LT may break the teeth UT and LT or periodontal ligaments and destroy an alveolar bone. Also, the teeth UT and LT may be lost or periodontitis may develop. Namely, the teeth UT and LT may be damaged.

In addition, the force exerted by the masticatory movements may spread to the muscles, ligaments, and TMJ around the oral cavity having the oral appliance 100 according to the exemplary embodiment of the present disclosure installed therein, damaging the muscles and ligaments. As a result, malocclusion or a TMJ disorder, as along with pain, may occur.

Meanwhile, if the gap D1 between the lower teeth recess 400 and the teeth LT of the lower jaw LJ in the vertical direction exceeds 2 mm, a distance between the teeth UT of the upper jaw UJ and the teeth LT of the lower jaw may be greater than 4 mm, the natural gap.

Thus, the related muscles and ligaments may be burdened and stiffened. Also, pain, malocclusion, and a TMJ disorder may occur.

Therefore, the gap D1 between the lower teeth recess 400 and the teeth LT of the lower jaw LJ in the vertical direction ranging from 0.5 mm to 2 mm is appropriate.

Meanwhile, the gap D2 between the lower teeth recess 400 and the teeth LT of the lower jaw LJ in the horizontal direction may range from 0.3 mm to 0.5 mm.

Also, the lower teeth recess 400 may be spaced apart from the teeth LT of the lower jaw LJ on both sides thereof. As discussed above, since the gap D2 between the lower teeth recess 400 and the teeth LT of the lower jaw LJ in the horizontal direction on one side of the lower teeth recess 400 ranges from 0.3 mm to 0.5 mm, a total gap in both sides may range from 0.6 mm to 1.0 mm.

If the gap D2 between the lower teeth recess 400 and the teeth LT of the lower jaw LJ in the horizontal direction on one side of the lower teeth recess 400 is smaller than 0.3 mm, the teeth LT of the lower jaw LJ may occasionally be fixed to the lower teeth recess 400 through friction. Also, the teeth LT of the lower jaw LJ may be hindered from being released and moving downwards from the lower teeth recess 400, when needed.

Thus, the related muscles and ligaments stiffened due to the forward movement of the lower jaw LJ may not be relaxed, causing pain, discomfort, malocclusion, or a TMJ disorder.

Meanwhile, when the gap D2 between the lower teeth recess 400 and the teeth LT of the lower jaw LJ in the horizontal direction on one side of the lower teeth recess 400 exceeds 0.5 mm, the teeth LT of the lower jaw LJ may be freely moved within the lower teeth recess 400, but the teeth LT of the lower jaw LJ may be completely released and separated from the lower teeth recess 400.

Thus, the lower jaw LJ may not be maintained in a state in which it is placed forward by a predetermined distance, losing the ability to prevent snoring or sleep apnea.

Therefore, the gap D2 between the lower teeth recess 400 and the teeth LT of the lower jaw LJ in the horizontal direction on one side of the lower teeth recess 400 ranging from 0.3 mm to 0.5 mm is appropriate.

The range of the gaps D1 and D2 between the lower teeth recess 400 and the teeth LT of the lower jaw LJ in the vertical or horizontal direction may be modified to be individually applied by adding or subtracting a value from a reference value of a portion or entirety of the lower teeth recess 400 according to a tilt angle of the occlusal plane, a degree of protrusion of teeth, an inclination state of teeth in forward/backward and inward/outward directions, or a degree of teeth deviating from the overall teeth arrangement, of each of the users.

Also, as illustrated in FIG. 4, the lower teeth recess 400 may be spaced apart from the teeth LT of the lower jaw LJ in the horizontal direction forwardly and backwardly. In addition, the lower teeth recess 400 may also be spaced apart from the teeth LT of the lower jaw LJ in the horizontal direction left and right.

As illustrated in FIGS. 1 and 2, the guide part 500 may extend downwards from the body 200. For example, the guide part 500 may extend downwards by 3 mm to 5 mm. However, the length of the guide part 500 extending from the body 200 may not be particularly limited and any length may be applied according to a state of an oral cavity of individuals.

As described above, as the teeth LT of the lower jaw LJ move downwards from the lower teeth recess 400 to relax the related muscles and ligaments stiffened as the lower jaw LJ moves forward and maintained in the state of being ahead by a predetermined distance, the lower jaw LJ may also be moved downwards by a predetermined distance naturally.

Such movement of the lower jaw LJ may be stopped when the guide part 500 comes into contact with the lingual mucous membrane of the lower jaw LJ, and the lower jaw LJ may be maintained in that state.

When the lower jaw LJ is moved or maintained in a moved state to relax the stiffened muscles and ligaments, other normal muscles or ligaments may be stiffened. In order to relax the other stiffened muscles and ligaments, the lower jaw LJ may be naturally moved upwards as illustrated in FIG. 5, and the teeth LT of the lower jaw LJ may be inserted into the lower teeth recess 400.

At this time, the guide part 500 may guide the teeth LT of the lower jaw LJ to be easily and properly inserted into the lower teeth recess 400.

To this end, the guide part 500 may extend from an inner side of the body 200 as illustrated in FIGS. 1 and 2. Also, the guide part 500 may extend to cover a portion of a gum part of the molars among the teeth LT of the lower jaw LJ.

However, the shape of the guide part 500 is not particularly limited and the guide part 500 may have any shape as long as it has the aforementioned functions.

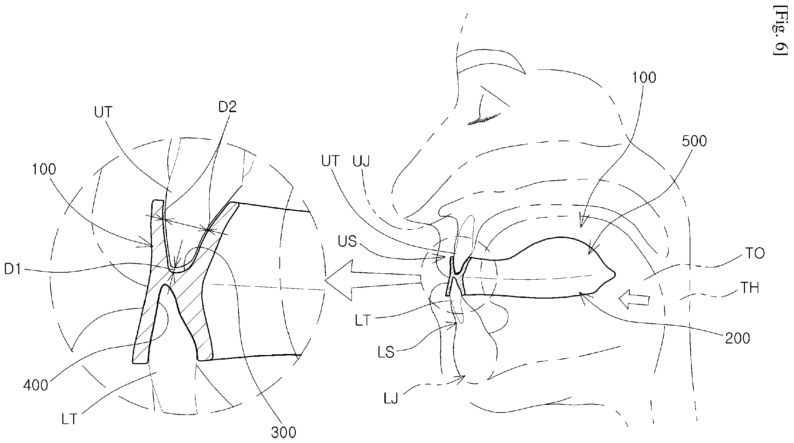

FIG. 6 is a cross-sectional view and a partially enlarged cross-sectional view illustrating a wearing state of the oral appliance according to a second exemplary embodiment of the present disclosure.

The oral appliance 100 according to the second exemplary embodiment of the present disclosure is different from that of the first exemplary embodiment described above with reference to FIGS. 1 through 5 in that the teeth LT of the lower jaw LJ is fixed in the lower teeth recess 400 through friction, the upper teeth recess 300 is spaced apart from the teeth UT of the upper jaw UJ at predetermined intervals D1 and D2 in vertical and horizontal directions, and the guide part 500 extends upwards from the body 200.

Thus, different parts and components will mainly be described, and the other components may simply refer back to the above descriptions with reference to FIGS. 1 through 5.

As illustrated in FIG. 6, in the oral appliance 100 according to the second exemplary embodiment of the present disclosure, the teeth UT of the lower jaw LJ may be fixed in the lower teeth recess 400. Namely, the lower teeth recess 400 may be formed to correspond to the teeth LT of the lower jaw LJ such that there is no gap between the lower teeth recess 400 and the teeth LT of the lower jaw LJ.

Thus, the teeth LT of the lower jaw LJ may be tightly inserted into the lower teeth recess 400 and fixed through friction. Also, the teeth LT of the lower jaw LJ may not be separated from the lower teeth recess 400 unless external force greater than frictional force between the lower teeth recess 400 and the teeth LT of the lower jaw LJ is applied.

The upper teeth recess 300 may be spaced apart from the teeth LT of the upper jaw UJ at predetermined intervals D1 and D2 in the vertical and horizontal directions.

The guide part 500 may extend upwards from the body 200.

Through this configuration, as the teeth LT of the upper jaw UJ are released from the upper teeth recess 300 and relatively moved in order to relax related muscles and ligaments stiffened due to a forward movement of the lower jaw LJ, the lower jaw LJ may naturally move downwards together with the oral appliance 100 according to the present exemplary embodiment.

While the lower jaw LJ is moving downwards together with the oral appliance 100, if the guide part 500 comes into contact with the molars among the teeth LT of the upper jaw UJ released from the upper teeth recess 300, the lower jaw LJ may stop moving and be maintained in the same state.

In this state, in order to relax other stiffened muscles and ligaments, the lower jaw LJ may naturally move upwards together with the oral appliance 100 according to the present exemplary embodiment to the position, namely, to the position prior to being released, in which the teeth LT of the upper jaw UJ are inserted into the upper teeth recess 300 and the lower jaw LJ moves forward. That is, the lower jaw LJ may be returned.

At this time, the guide part 500 may guide the teeth LT of the upper jaw UJ to be easily and properly inserted into the upper teeth recess 300.

The reciprocating movement of the lower jaw LJ may be repeated while the user wearing the oral appliance 100 according to the exemplary embodiment of the present disclosure is sleeping.

Thus, as described above, the related muscles and ligaments stiffened due to the forward movement of the lower jaw and the movement of the lower jaw may be relaxed, and thus, even though the user wears the oral appliance, pain, malocclusion, or a TMJ disorder may not occur. Also, since air flow through the airway is facilitated, rhinitis or rhinosinusitis may be mitigated.

Meanwhile, the gap D1 between the upper teeth recess 300 and the teeth LT of the upper jaw UJ in the vertical direction may range from 0.5 mm to 2 mm. Also, the gap D2 between the upper teeth recess 300 and the teeth LT of the upper jaw UJ in the horizontal direction in one side thereof may range from 0.3 mm to 0.5 mm.

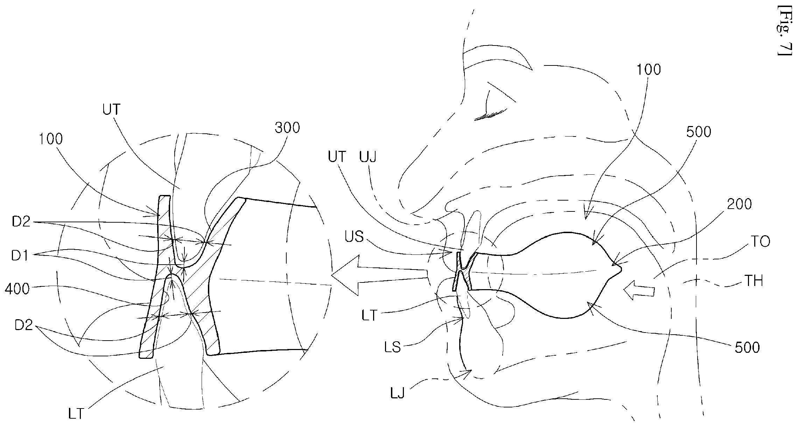

FIG. 7 is a cross-sectional view and a partially enlarged cross-sectional view illustrating a wearing state of an oral appliance according to a third exemplary embodiment of the present disclosure.

The oral appliance 100 according to the third exemplary embodiment of the present disclosure is different from that of the first exemplary embodiment, described above with reference to FIGS. 1 through 5, in that the upper teeth recess 300 is spaced apart from the teeth UT of the upper jaw UJ at predetermined intervals D1 and D2 in vertical and horizontal directions, the lower teeth recess 400 is spaced apart from the teeth UT of the lower jaw LJ at predetermined intervals D1 and D2 in vertical and horizontal directions, and the guide part 500 extends downwards and upwards from the body 200.

Thus, different parts and components will mainly be described, and the other components may simply refer back to the above descriptions with reference to FIGS. 1 through 5.

As illustrated in FIG. 7, in the oral appliance 100 according to the third exemplary embodiment of the present disclosure, the upper teeth recess 300 may be spaced apart from the teeth UT of the upper jaw UJ at predetermined intervals D1 and D2 in the vertical and horizontal directions, and lower teeth recess 400 may be spaced apart from the teeth LT of the lower jaw LJ at predetermined intervals D1 and D2 in the vertical and horizontal directions.

Also, the guide part 500 may extend both upwards and downwards by a predetermined distance.

The oral appliance 100 according to the third exemplary embodiment of the present disclosure may be used in a case in which a state of the teeth arrangements US and LS of the upper and lower jaws UJ and LJ or a state of the teeth UT and LT included therein is so poor, such that the teeth may be lost if fixed to the upper teeth recess 300 or the lower teeth recess 400 through friction, in a case in which a length of the crowns of the upper and lower jaws is too short to obtain sufficient frictional force.

In this configuration, when any one of the teeth UT of the upper jaw UJ and the teeth LT of the lower jaw LJ is more tightly attached to the upper teeth recess 300 or the lower teeth recess 400 according to posture during sleep, a side less tightly attached may be moved within the upper teeth recess 300 or the lower teeth recess 400. Thus, the lower jaw LJ may be naturally moved to relax the stiffened muscles and ligaments.

For example, if the teeth UT of the upper jaw UJ are more tightly attached to the upper teeth recess 300, the lower jaw LJ may perform a reciprocating movement in a manner identical to that of the oral appliance 100 according to the first exemplary embodiment of the present disclosure as described above, and if the teeth UT of the lower jaw LJ are more tightly attached to the lower teeth recess 400, the lower jaw LJ may perform a reciprocating movement in a manner identical to that of the oral appliance 100 according to the second exemplary embodiment of the present disclosure as described above.

The reciprocating movement of the lower jaw LJ may be repeated while the user wearing the oral appliance 100 according to the exemplary embodiment of the present disclosure is sleeping.

Thus, as described above, the related muscles and ligaments stiffened due to the forward movement of the lower jaw and the other movement of the lower jaw may be relaxed, and thus, even though the user wears the oral appliance, pain, malocclusion, or a TMJ disorder may not occur. Also, since air flow through the airway is facilitated, rhinitis or rhinosinusitis may be mitigated.

In this case, the gap D1 between the upper teeth recess 300 and the teeth LT of the upper jaw UJ in the vertical direction and the gap D2 between the lower teeth recess 400 and the teeth LT of the lower jaw LJ in the vertical direction may range from 0.5 mm to 2 mm.

Also, the gap D2 between the upper teeth recess 300 and the teeth LT of the upper jaw UJ in the horizontal direction in one side thereof, and the gap D2 between the lower teeth recess 400 and the teeth LT of the lower jaw LJ in the horizontal direction in one side thereof may range from 0.3 mm to 0.5 mm.

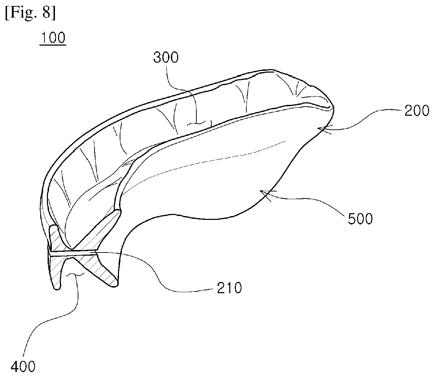

FIG. 8 is a perspective cross-sectional view illustrating an oral appliance according to a further example of the present disclosure which does not fall under the scope of the claimed invention.

The oral appliance 100 according to this example is different from that of the first exemplary embodiment described above with reference to FIGS. 1 through 5, in that a vent hole 210 is formed in the body 200 of the oral appliance 100.

Thus, different parts and components will mainly be described, and the other components may simply refer back to the above descriptions with reference to FIGS. 1 through 5.

As illustrated in FIG. 8, in the oral appliance 100, the vent hole 210 may be formed in the body 200. The vent hole 210 may be formed in a central portion of the body 200 between the upper teeth recess 300 and the lower teeth recess 400.

Thus, the user may breathe through his or her mouth through the vent hole 210 of the body 200 while wearing the oral appliance 100.

As described above, in the case of using the oral appliance according to the exemplary embodiment of the present disclosure, even if a user wears the oral appliance, a lower jaw may naturally move, and related muscles and ligaments stiffened due to a forward movement of the lower jaw may be relaxed as the lower jaw is allowed to move. In addition, although a user wears the oral appliance, pain, malocclusion, or a TMJ disorder may not occur, and since air flow through an airway is facilitated, rhinitis or rhinosinusitis may be mitigated.

The oral appliance as described above is not limited in its application to the configurations of the foregoing exemplary embodiments, but the entirety or a portion of the exemplary embodiments can be selectively combined to configure various modifications. The scope of the invention is defined by the appended claims.

## Claims

1. An oral appliance comprising:
a body (200) having a shape corresponding to a teeth arrangement;
an upper teeth recess (300) formed in an upper portion of the body (200) to allow teeth included in the teeth arrangement (US) of an upper jaw (UJ) to be inserted therein; and
a lower teeth recess (400) formed in a lower portion of the body (200) to allow teeth included in the teeth arrangement (LS) of a lower jaw (LJ) to be inserted therein and allowing the lower jaw (LJ) to move forward by a predetermined distance
**characterized in that**
in use, the teeth of the upper jaw (UJ) are fixed in the upper teeth recess (300) through friction, and the lower teeth recess (400) is spaced apart from the teeth of the lower jaw (LJ) at predetermined intervals (D1, D2) in vertical and horizontal directions.

2. The oral appliance of claim 1, further comprising a guide part (500) extending from the body (200) in at least one or more of upward and downward directions by a predetermined distance to thereby guide teeth of the lower jaw (LJ) or upper jaw (UJ) to be inserted into the upper teeth recess (300) or the lower teeth recess (400).

3. The oral appliance of claim 2, wherein the guide part (500) extends from an inner side of the body (200).

4. The oral appliance of claim 2, wherein the guide part (500) is adapted to extend to cover, in use, at least a portion of a gum part of molars among the teeth.

5. The oral appliance of claim 1, wherein, in use, a gap between the lower teeth recess (400) and the teeth of the lower jaw (LJ) in the vertical direction ranges from 0.5 mm to 2 mm.

6. The oral appliance of claim 1, wherein in use, a gap between the lower teeth recess (400) and the teeth of the lower jaw (LJ) in the horizontal direction in one side thereof ranges from 0.3 mm to 0.5 mm.

7. An oral appliance comprising:
a body (200) having a shape corresponding to a teeth arrangement;
an upper teeth recess (300) formed in an upper portion of the body (200) to allow teeth included in the teeth arrangement (US) of an upper jaw (UJ) to be inserted therein; and
a lower teeth recess (400) formed in a lower portion of the body (200) to allow teeth included in the teeth arrangement (LS) of a lower jaw (LJ) to be inserted therein and allowing the lower jaw (LJ) to move forward by a predetermined distance
**characterized in that**
in use, the teeth of the lower jaw (LJ) are fixed in the lower teeth recess (400) through friction, and the upper teeth recess (300) is spaced apart from the teeth of the upper jaw (UJ) at predetermined intervals (D1, D2) in vertical and horizontal directions.

8. The oral appliance of claim 7, wherein in use, a gap between the upper teeth recess (300) and the teeth of the upper jaw (UJ) in the vertical direction ranges from 0.5 mm to 2 mm.

9. The oral appliance of claim 7, wherein, in use, a gap between the upper teeth recess (300) and the teeth of the upper jaw (UJ) in the horizontal direction in one side thereof ranges from 0.3 mm to 0.5 mm.

10. An oral appliance comprising:
a body (200) having a shape corresponding to a teeth arrangement;
an upper teeth recess (300) formed in an upper portion of the body (200) to allow teeth included in the teeth arrangement (US) of an upper jaw (UJ) to be inserted therein; and
a lower teeth recess (400) formed in a lower portion of the body (200) to allow teeth included in the teeth arrangement (LS) of a lower jaw (LJ) to be inserted therein and allowing the lower jaw (LJ) to move forward by a predetermined distance
**characterized in that**
in use, the upper teeth recess (300) is spaced apart from the teeth of the upper jaw (UJ) at a predetermined interval in vertical and horizontal directions, and the lower teeth recess (400) is spaced apart from the teeth of the lower jaw (LJ) at a predetermined interval (D1, D2) in vertical and horizontal directions.

11. The oral appliance of claim 10, wherein in use, a gap between the upper teeth recess (300) and the teeth of the upper jaw (UJ) in the vertical direction and a gap between the lower teeth recess (400) and the teeth of the lower jaw (LJ) in the vertical direction range from 0.5 mm to 2 mm, and
a gap between the upper teeth recess (300) and the teeth of the upper jaw (UJ) in the horizontal direction in one side thereof and a gap between the lower teeth recess (400) and the teeth of the lower jaw (LJ) in the horizontal direction in one side thereof range from 0.3 mm to 0.5 mm.

## Patentansprüche

1. Orale Vorrichtung, umfassend:
einen Körper (200) mit einer Form, die einer Zahnanordnung entspricht;
eine obere Zahnaussparung (300), die in einem oberen Abschnitt des Körpers (200) ausgebildet ist, um es zu ermöglichen, dass Zähne, die in der Zahnanordnung (US) eines Oberkiefers (UJ) enthalten sind, darin eingesetzt werden können; und
eine untere Zahnaussparung (400), die in einem unteren Abschnitt des Körpers (200) ausgebildet ist, um es zu ermöglichen, dass Zähne, die in der Zahnanordnung (LS) eines Unterkiefers (LJ) enthalten sind, darin eingesetzt werden können und dass sich der Unterkiefer (LJ) um einen vorbestimmten Abstand nach vorne bewegen kann.
**dadurch gekennzeichnet, dass**
im Gebrauch die Zähne des Oberkiefers (UJ) durch Reibung in der oberen Zahnaussparung (300) fixiert sind und die untere Zahnaussparung (400) von den Zähnen des Unterkiefers (LJ) in vorbestimmten Abständen (D1, D2) in vertikaler und horizontaler Richtung beabstandet ist.

2. Orale Vorrichtung nach Anspruch 1, des Weiteren umfassend ein Führungsteil (500), das sich von dem Körper (200) in mindestens einer oder mehreren Auf- und Abwärtsrichtungen um einen vorbestimmten Abstand erstreckt, um dadurch Zähne des Unterkiefers (LJ) oder des Oberkiefers (UJ) zu führen, die in die obere Zahnaussparung (300) oder die untere Zahnaussparung (400) einzusetzen sind.

3. Orale Vorrichtung nach Anspruch 2, wobei sich das Führungsteil (500) von einer Innenseite des Körpers (200) erstreckt.

4. Orale Vorrichtung nach Anspruch 2, wobei das Führungsteil (500) so angepasst ist, dass es sich erstreckt, um im Gebrauch mindestens einen Abschnitt eines Gummiteils von Molaren zwischen den Zähnen abzudecken.

5. Orale Vorrichtung nach Anspruch 1, wobei im Gebrauch ein Spalt zwischen der unteren Zahnaussparung (400) und den Zähnen des Unterkiefers (LJ) in vertikaler Richtung im Bereich von 0,5 mm bis 2 mm liegt.

6. Orale Vorrichtung nach Anspruch 1, wobei im Gebrauch ein Spalt zwischen der unteren Zahnaussparung (400) und den Zähnen des Unterkiefers (LJ) in horizontaler Richtung in einer Seite hiervon im Bereich von 0,3 mm bis 0,5 mm liegt.

7. Orale Vorrichtung, umfassend:
einen Körper (200) mit einer Form, die einer Zahnanordnung entspricht;
eine obere Zahnaussparung (300), die in einem oberen Abschnitt des Körpers (200) ausgebildet ist, um es zu ermöglichen, dass Zähne, die in der Zahnanordnung (US) eines Oberkiefers (UJ) enthalten sind, darin eingesetzt werden können; und
eine untere Zahnaussparung (400), die in einem unteren Abschnitt des Körpers (200) ausgebildet ist, um es zu ermöglichen, dass Zähne, die in der Zahnanordnung (LS) eines Unterkiefers (LJ) enthalten sind, darin eingesetzt werden können und dass sich der Unterkiefer (LJ) um einen vorbestimmten Abstand nach vorne bewegen kann.
**dadurch gekennzeichnet, dass**
im Gebrauch die Zähne des Unterkiefers (LJ) durch Reibung in der unteren Zahnaussparung (400) fixiert sind und die obere Zahnaussparung (300) von den Zähnen des Oberkiefers (UJ) in vorbestimmten Abständen (D1, D2) in vertikaler und horizontaler Richtung beabstandet ist.

8. Orale Vorrichtung nach Anspruch 7, wobei im Gebrauch ein Spalt zwischen der oberen Zahnaussparung (300) und den Zähnen des Oberkiefers (UJ) in vertikaler Richtung im Bereich von 0,5 mm bis 2 mm liegt.

9. Orale Vorrichtung nach Anspruch 7, wobei im Gebrauch ein Spalt zwischen der oberen Zahnaussparung (300) und den Zähnen des Oberkiefers (UJ) in horizontaler Richtung in einer Seite hiervon im Bereich von 0,3 mm bis 0,5 mm liegt.

10. Orale Vorrichtung, umfassend:
einen Körper (200) mit einer Form, die einer Zahnanordnung entspricht;
eine obere Zahnaussparung (300), die in einem oberen Abschnitt des Körpers (200) ausgebildet ist, um es zu ermöglichen, dass Zähne, die in der Zahnanordnung (US) eines Oberkiefers (UJ) enthalten sind, darin eingesetzt werden können; und
eine untere Zahnaussparung (400), die in einem unteren Abschnitt des Körpers (200) ausgebildet ist, um es zu ermöglichen, dass Zähne, die in der Zahnanordnung (LS) eines Unterkiefers (LJ) enthalten sind, darin eingesetzt werden können und dass sich der Unterkiefer (LJ) um einen vorbestimmten Abstand nach vorne bewegen kann.
**dadurch gekennzeichnet, dass**
im Gebrauch die obere Zahnaussparung (300) von den Zähnen des Oberkiefers (UJ) in einem vorbestimmten Abstand in vertikalen und horizontalen Richtungen beabstandet ist, und die untere Zahnaussparung (400) von den Zähnen des Unterkiefers (LJ) in einem vorbestimmten Abstand (D1, D2) in vertikalen und horizontalen Richtungen beabstandet ist.

11. Orale Vorrichtung nach Anspruch 10, wobei im Gebrauch ein Spalt zwischen der oberen Zahnaussparung (300) und den Zähnen des Oberkiefers (UJ) in vertikaler Richtung und ein Spalt zwischen der unteren Zahnaussparung (400) und den Zähnen des Unterkiefers (LJ) in vertikaler Richtung im Bereich von 0,5 mm bis 2 mm liegen, und
ein Spalt zwischen der oberen Zahnaussparung (300) und den Zähnen des Oberkiefers (UJ) in horizontaler Richtung in einer Seite hiervon und ein Spalt zwischen der unteren Zahnaussparung (400) und den Zähnen des Unterkiefers (LJ) in horizontaler Richtung in einer Seite hiervon im Bereich von 0,3 mm bis 0,5 mm liegen.

## Revendications

1. Appareil oral comprenant:
un corps (200) ayant une forme correspondant à un agencement de dents;
un évidement de dents supérieures (300) formé dans une partie supérieure du corps (200) pour permettre aux dents incluses dans l'arrangement de dents (US) d'une mâchoire supérieure (UJ) d'y être insérées; et
un évidement de dents inférieures (400) formé dans une partie inférieure du corps (200) pour permettre aux dents incluses dans l'arrangement de dents (LS) d'une mâchoire inférieure (LJ) d'y être insérées et permettre à la mâchoire inférieure (LJ) d'avancer d'une distance prédéterminée
**caractérisé en ce que**
lors de l'utilisation les dents de la mâchoire supérieure (UJ) sont fixées dans l'évidement de dents supérieures (300) par frottement, et l'évidement de dents inférieures (400) est espacé des dents de la mâchoire inférieure (LJ) à des intervalles prédéterminés (D1, D2) dans les directions verticale et horizontale.

2. Appareil oral selon la revendication 1, comprenant en outre une partie de guidage (500) s'étendant à partir du corps (200) dans au moins une ou plusieurs directions ascendantes et descendantes d'une distance prédéterminée pour guider ainsi les dents de la mâchoire inférieure (LJ) ou supérieure (UJ) à insérer dans l'évidement de dents supérieures (300) ou l'évidement de dents inférieures (400).

3. Appareil oral selon la revendication 2, dans lequel la partie de guidage (500) s'étend depuis un côté intérieur du corps (200).

4. Appareil oral selon la revendication 2, dans lequel la partie de guidage (500) est adaptée pour couvrir, lors de l'utilisation, au moins une partie d'une partie d'une partie de gencive de molaires parmi les dents.

5. Appareil oral selon la revendication 1, dans lequel, lors de l'utilisation, un espace entre l'évidement des dents inférieures (400) et les dents de la mâchoire inférieure (LJ) dans la direction verticale va de 0,5 mm à 2 mm.

6. Appareil oral selon la revendication 1, dans lequel, lors de l'utilisation, un espace entre l'évidement des dents inférieures (400) et les dents de la mâchoire inférieure (LJ) dans la direction horizontale dans un de ses côtés est compris entre 0,3 mm et 0,5 mm.

7. Appareil oral comprenant:
un corps (200) ayant une forme correspondant à un agencement de dents;
un évidement de dents supérieures (300) formé dans une partie supérieure du corps (200) pour permettre aux dents incluses dans l'arrangement de dents (US) d'une mâchoire supérieure (UJ) d'y être insérées; et
un évidement de dents inférieures (400) formé dans une partie inférieure du corps (200) pour permettre aux dents incluses dans l'arrangement de dents (LS) d'une mâchoire inférieure (LJ) d'y être insérées et permettre à la mâchoire inférieure (LJ) d'avancer d'une distance prédéterminée
**caractérisé en ce que**
lors de l'utilisation, les dents de la mâchoire inférieure (LJ) sont fixées dans l'évidement de dents inférieures (400) par frottement, et l'évidement de dents supérieures (300) est espacé des dents de la mâchoire supérieure (UJ) à des intervalles prédéterminés (D1, D2) dans les directions verticale et horizontale.

8. Appareil oral selon la revendication 7, dans lequel, lors de l'utilisation, un espace entre l'évidement des dents supérieures (300) et les dents de la mâchoire supérieure (UJ) dans la direction verticale varie de 0,5 mm à 2 mm.

9. Appareil oral selon la revendication 7, dans lequel, lors de l'utilisation, un espace entre l'évidement des dents supérieures (300) et les dents de la mâchoire supérieure (UJ) dans la direction horizontale dans un de ses côtés est compris entre 0,3 mm et 0,5 mm.

10. Appareil oral comprenant:
un corps (200) ayant une forme correspondant à un agencement de dents;
un évidement de dents supérieures (300) formé dans une partie supérieure du corps (200) pour permettre aux dents incluses dans l'arrangement de dents (US) d'une mâchoire supérieure (UJ) d'y être insérées; et
un évidement de dents inférieures (400) formé dans une partie inférieure du corps (200) pour permettre aux dents incluses dans l'arrangement de dents (LS) d'une mâchoire inférieure (LJ) d'y être insérées et permettre à la mâchoire inférieure (LJ) d'avancer d'une distance prédéterminée
**caractérisé en ce que**
lors de l'utilisation, l'évidement de dents supérieures (300) est espacé des dents de la mâchoire supérieure (UJ) à un intervalle prédéterminé dans les directions verticale et horizontale, et l'évidement de dents inférieures (400) est espacé des dents de la mâchoire inférieure (LJ) à un intervalle prédéterminé (D1, D2) dans les directions verticale et horizontale.

11. Appareil oral selon la revendication 10, dans lequel, lors de l'utilisation, un espace entre l'évidement de dents supérieures (300) et les dents de la mâchoire supérieure (UJ) dans la direction verticale et un espace entre l'évidement de dents inférieures (400) et les dents de la mâchoire inférieure (LJ) dans la direction verticale sont de 0,5 mm à 2 mm, et
un espace entre l'évidement de dents supérieures (300) et les dents de la mâchoire supérieure (UJ) dans la direction horizontale dans un de ses côtés et un espace entre l'évidement de dents inférieures (400) et les dents de la mâchoire inférieure (LJ) dans la direction horizontale dans un de ses côtés varient de 0,3 mm à 0,5 mm.
